(19) Europäisches Patentamt
European Patent Office
Office européen des brevets

(11) Veröffentlichungsnummer : **0 346 707 B1**

# (12) EUROPÄISCHE PATENTSCHRIFT

(45) Veröffentlichungstag der Patentschrift : **17.02.93 Patentblatt 93/07**

(51) Int. Cl.$^5$ : **A61K 6/08**

(21) Anmeldenummer : **89110092.7**

(22) Anmeldetag : **03.06.89**

(54) **Dentalwerkstoffe.**

(30) Priorität : **16.06.88 DE 3820498**

(43) Veröffentlichungstag der Anmeldung : **20.12.89 Patentblatt 89/51**

(45) Bekanntmachung des Hinweises auf die Patenterteilung : **17.02.93 Patentblatt 93/07**

(84) Benannte Vertragsstaaten : **AT BE CH DE ES FR GB GR IT LI LU NL SE**

(56) Entgegenhaltungen :
**EP-A- 0 270 915**
**US-A- 4 396 377**

(73) Patentinhaber : **BAYER AG**
**W-5090 Leverkusen 1 Bayerwerk (DE)**

(72) Erfinder : **Podszun, Wolfgang, Dr.**
**Roggendorfstrasse 55**
**W-5000 Köln 80 (DE)**
Erfinder : **Bley, Fritjof, Dipl.-Ing.**
**Hohbuchweg 21**
**W-8991 Achberg-Liebenweiler (DE)**

**EP 0 346 707 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft Dentalwerkstoffe, ihre Herstellung und ihre Verwendung.

Dentalwerkstoffe können beispielsweise zur Herstellung künstlicher Zähne, Kronen, Brücken, Inlays, Onlays, Zahnfüllungen und Zahnlacke eingesetzt werden.

Es ist bekannt, Dentalwerkstoffe auf Basis von polymeren (Meth)-Acrylaten herzustellen. So stellt man beispielsweise Werkstoffe her, die Polymethylmethacrylat-Perlpolymerisate als Pulverkomponente und Mischungen aus Methylmethacrylat und Ethylendimethacrylat enthalten; die Mischungen härten im allgemeinen durch radikalische Polymerisation unter Formgebung aus (Ullmann' 8 Encyclopedia of Industrial Chemistry, Fifth Edition, Volume A8, S, 277 ff. VCH Verlagsgesellschaft m.b.H., Weinheim 1987).

In der WO 82/02554 und in US-A-4,396,377 werden Dentalwerkstoffe beschrieben, die neben unvernetztem Polymethylmethacrylat ein vernetztes Polymethylmethacrylat in Form eines Perlpolymerisats als Füllstoff enthalten, Diese Werkstoffe sind nach dem Prinzip des "Interpenetrating-Polymer-Network" (IPN) aufgebaut. IPN-systeme sind bekannt (J, Polymer, Science 12, 141 (1977), J. Polym. Science 16, 583 (1978)).

Die aus der WO 82/02556 und US-A-4,396,377 bekannten Werkstoffe weisen keine ausreichenden mechanischen Eigenschaften auf und lassen sich nicht in beliebig dünnen Schichten (z.B. bei Zahnlacken) verarbeiten.

Es wurden Dentalwerkstoffer enthaltend

a) 5 bis 35 Gew.-Teile eines Füllstoffs, bestehend aus polymeren vernetzten (Meth)-Acrylaten mit einer Teilchengröße im Bereich von 0,01 bis 10 µm, einem Quellungsgrad von 50 bis 2000 Gew.-% und einem Vernetzungsgrad von 50 bis 100 Gew.-%, jeweils bezogen auf das Polymer,

b) 40 bis 90 Gew.-Teile (Meth)-Acrylate, die Vernetzungen ausbilden können,

c) 0 bis 40 Gew.-Teile (Meth)-Acrylate, die keine Vernetzungen ausbilden können und

d) 0,1 bis 10 Gew.-Teile eines oder mehrere Additive,

gefunden.

Die erfindungsgemäßen Dentalwerkstoffe zeichnen sich überraschenderweise durch eine große Härte und Steifigkeit aus. Sie lassen sich zu sehr dünnen Schichten verarbeiten.

(Meth)acrylate im Rahmen der vorliegenden Erfindung sind Ester der Acrylsäure und/oder der Methacrylsäure. Bevorzugt sind Ester der Methacrylsäure.

### Komponente (a)

Füllstoffe im Rahmen der Erfindung sind polymere vernetzte (Meth)-Acrylate mit einer Teilchengröße im Bereich von 0,01 - 10 µm,
die einen Quellungsgrad von 50 bis 2000 Gew.-% und einen Vernetzungsgrad von 50 bis 100 Gew.-%, jeweils bezogen auf das Polymer, aufweisen.

Als Vernetzungsgrad wird hier der Anteil an Methacrylsäureestern, die Vernetzungen bezogen auf das Polymer ausbilden können, definiert.

Monomere (Meth)-Acrylate der Füllstoffe, die Vernetzungen ausbilden, sind (Meth)-Acrylate mit 2 oder mehr, bevorzugt 2 bis 4, polymerisierbare Doppelbindungen im Molekül.

Als monomere (Meth)-Acrylate, die Vernetzungen ausbilden, seien beispielsweise genannt:

Ethylenglykoldimethacrylat, Diethylenglykoldimethacrylat, Triethylenglykoldimethacrylat, Glycerindimethacrylat, Glycerintrimethacrylat, Trimethylolpropantrimethacrylat, Pentaerythritdimethacrylat, Pentaerythrittrimethacrylat, Pentaerythrittetramethacrylat,
Derivate des Bisphenol A, wie Bisphenol-A-dimethacrylat und Bisphenol-A-diglycidyldimethacrylat,
Urethanmethacrylate, die durch Umsetzung von Diisocyanaten und Hydroxyalkylmethacrylaten hergestellt werden können, wie

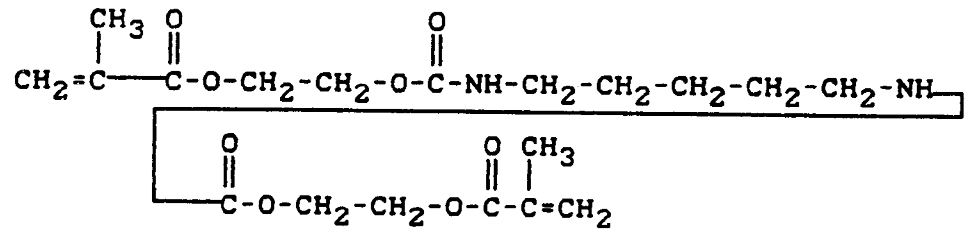

Umsetzungsprodukte aus Polyolen, Diisocyanaten und Hydroxyalkylmethacrylaten (DE-A 37 03 080, DE-A 37 03 130 und DE-A 37 03 120), wie z.B.

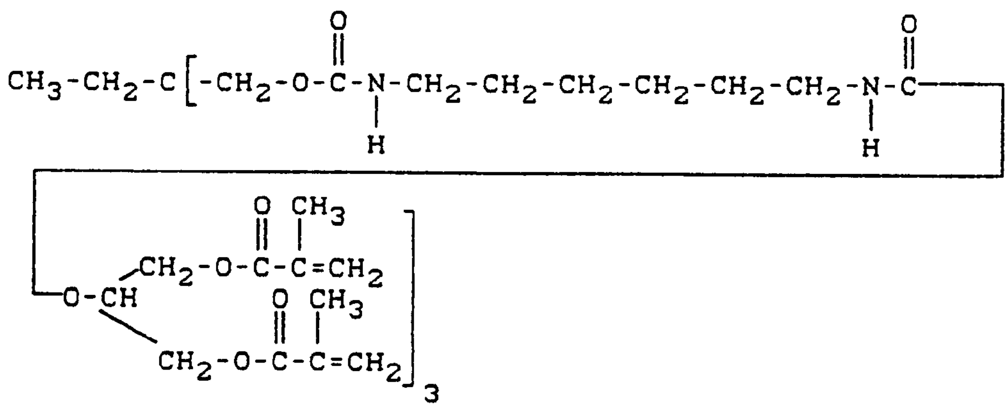

Bevorzugt werden monomere (Meth)-Acrylate, die Vernetzungen ausbilden, wie:

Ethylenglycoldimethacrylat, Diethylenglycoldimethacrylat, Triethylenglycolidmethacrylat und Glycerindimethacrylat.

Als monomere (Meth)-Acrylate der Füllstoffe, die keine Vernetzungen ausbilden, seien beispielsweise genannt:

$C_1$-$C_{12}$-, bevorzugt $C_1$-$C_4$-Alkylmethacrylate, wie Methylmethacrylat, Ethylmethacrylat, n-Propylmethacrylat, iso-Propylmethacrylat, n-Butylmethacrylat, t-Butylmethacrylat,
Hydroxyalkyl($C_1$-$C_4$)methacrylate, wie 2-Hydroxyethylmethacrylat, 2-Hydroxypropylmethacrylat, Diethylenglykolmonomethacrylat, Triethylenglykolmonomethacrylat, Alkoxy($C_1$-$C_4$)ethylmethacrylat, wie 2-Methoxyethylmethacrylat, 3-Methoxybutylmethacrylat und Ethyltriglykolmethacrylat.

Bevorzugte monomere (Meth)-Acrylate, die keine Vernetzungen ausbilden, sind beispielsweise:

Methylmethacrylat, Ethylmethacrylat, 2-Hydroxyethylmethacrylat.

Die monomeren (Meth)-Acrylate sind an sich bekannt und können beispielsweise durch Umsetzung von (Meth)Acrylsäurechlorid mit den entsprechenden Alkoholen hergestellt werden.

Es ist selbstverständlich möglich, daß die erfindungsgemäßen (Meth)-Acrylate der Füllstoffe mit weiteren Monomeren Copolymere bilden. Beispielsweise seien hier Copolymere mit Styrol, $\alpha$-Methylstyrol, Acrylnitril und Vinylacetat genannt. In diesen Fällen beträgt der Anteil des Comonomers 0 bis 40, bevorzugt 0 bis 20 Gew.-%, bezogen auf das Polymer.

Unter dem Quellungsgrad versteht man das Aufnahmevermögen der erfindungsgemäßen Poly(meth)acrylate an Flüssigkeit. Der Quellungsgrad wird gemessen durch das Aufnahmevermögen an Tetrahydrofuran bei 20°C. Die erfindungsgemäßen Poly(meth)acrylate weisen ein Quellungsvermögen von 50 bis 2000 Gew.-%, bevorzugt von 100 bis 1000 Gew.-%, bezogen auf das Polymer, auf.

Die erfindungsgemäß verwendeten Füllstoffe weisen einen mittleren Teilchendurchmesser von 0,01 bis 10 µm auf.

Bevorzugt weisen die erfindungsgemäßen Füllstoffe einen Gelgehalt von 5 bis 100 Gew.-%, bevorzugt von 95 bis 100 Gew.-%, bezogen auf das Polymer, auf. Im Rahmen der vorliegenden Erfindung versteht man unter dem Gelgehalt definitionsgemäß den Anteil des Polymers, der mit THF als Lösungsmittel bei 20°C nicht löslich ist. Der Gelgehalt ist eine Kenngröße für die tatsächlich eingetretene Vernetzung.

Die erfindungsgemäßen Füllstoffe weisen bevorzugt eine aktive Oberfläche von 20 bis 600 $m^2/g$, bevorzugt von 50 bis 300 $m^2/g$, gemessen nach der BET-Methode auf.

Erfindungsgemäße Füllstoffe mit einer Teilchengröße von ca. 5 - 10 µm lassen sich nach dem Verfahren der Suspensionspolymerisation synthetisieren.

Bevorzugte erfindungsgemäße Füllstoffe mit einer Teilchengröße von 0,01 bis 10 µm lassen sich dadurch herstellen, daß man als Monomere (Meth)-Acrylate, die Vernetzungen ausbilden, und gegebenenfalls (Meth)-Acrylate, die keine Vernetzungen ausbilden, und gegebenenfalls weitere Comonomere in Gegenwart eines organischen Lösungsmittels mit einem Löslichkeitsparameter von 8 bis 15 [$cal^{0,5}cm^{-1,5}$] polymerisiert, wobei der Monomeranteil der (Meth)-Acrylate, die Vernetzungen ausbilden, 50 bis 100 Gew.-% beträgt.

Organische Lösungsmittel können durch den sogenannten Löslichkeitsparameter definiert werden (H.G. Elias, Makromoleküle, S. 192-196 (1981)). Für das erfindungsgemäße Verfahren verwendet man Lösungsmittel mit einem Parameter von 8 bis 15 [$cal^{0,5}cm^{-1,5}$], bevorzugt von 8,5 bis 12 [$cal^{0,5}cm^{-1,5}$].

Beispielsweise seien die folgenden Lösungsmittel genannt:

Amylacetat, Tetrachlorethan, Toluol, Ethylacetat, Tetrahydrofuran, Benzol, Trichlormethan, Dichlormethan, Methylchlorid, Aceton, Butanon-2 und tert.-Butanol.

Die Menge des Lösungsmittels im Verhältnis zu den monomeren (Meth)-Acrylaten, liegt im Bereich von 1:1 bis 1:100, vorzugsweise 1:2 bis 1:20.

Die Durchführung des erfindungsgemäßen Polymerisationsverfahrens erfolgt im allgemeinen im Temperaturbereich von 50 bis 250°C, bevorzugt von 60 bis 150°C. Dabei kann die Polymerisation kontinuierlich oder diskontinuierlich durchgeführt werden.

Die Polymerisation wird im allgemeinen in Gegenwart von Initiatoren wie Sensibilisatoren oder Radikalbildnern durchgeführt.

Die Initiatoren werden im allgemeinen in einer Menge von 0,01 bis 3 Gew.-%, vorzugsweise 0,1 bis 1,5 Gew.-%, bezogen auf das Gesamtmonomere, eingesetzt.

Als Polymerisationsinitiatoren können beispielsweise Perverbindungen oder Radikale liefernde Azoverbindungen verwendet werden. Beispielsweise seien genannt:

Aliphatische Azodicarbonsäurederivate wie Azobisisobuttersäurenitril oder Azodicarbonsäureester, Peroxide wie Lauroylperoxid, Succinylperoxid, Dibenzoylperoxid, p-Chlorbenzoylperoxid, 2,4-Dichlorbenzoylperoxid, Peroxide wie Methylethylketonperoxid, Methylisobutylketonperoxid, Cyclohexanonperoxid, Acetylacetonperoxid, Alkylester von Persäuren wie tert.-Butylperpivalat, tert.-Butylperoctoat, tert.-Butylperbenzoat, tert.-Butylperisononat, Mono-tert.-Butylpermaleinat, tert.-Butylperacetat, Percarbonate wie Dicyclohexyl- und Diisopropylpercarbonat, Dialkylperoxide wie Di-tert.-Butylperoxid, Dicumylperoxid, Hydroperoxide wie tert.-Butyl- oder Cumolhydroperoxide, Isophthal-monopersäure oder Acetylcyclohexansulfonylperoxid.

Bei der erfindungsgemäßen Polymerisation entsteht im allgemeinen eine Suspension des Füllstoffs. Die Isolierung des Füllstoffs kann beispielsweise durch Verdampfen des Lösungsmittels, beispielsweise in einem Sprühtrocknungsprozeß, erfolgen.

Komponente (b)

(Meth)-Acrylsäureester, die Vernetzungen ausbilden können, enthalten im allgemeinen 2 oder mehr polymerisierbare aktive Gruppen, z.B. Doppelbindungen oder Isocyanatgruppen, im Molekül. Bevorzugt seien Ester der (Meth)-Acrylsäure mit 2 bis 5-wertigen Alkoholen mit 2 bis 30 Kohlenstoffatomen genannt. Besonders bevorzugt werden Epoxidmethacrylate und Urethanmethacrylate.

Beispielsweise seien (Meth)-Acrylsäureester der Formel

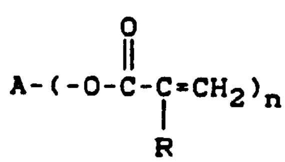

in der

A einen geradkettigen, verzweigten, cyclischen, aliphatischen, aromatischen oder gemischt aliphatisch-aromatischen Rest mit 2 bis 25 C-Atomen, der durch -O- oder NH-Brücken unterbrochen und mit Hydroxy, Oxy, Carboxy, Amino oder Halogen substituiert sein kann,

bedeutet,

R H oder Methyl bedeutet und

n für eine ganze Zahl von 2 bis 8, vorzugsweise 2 bis 4, steht, genannt.

Vorzugsweise seien Verbindungen der folgenden Formeln genannt:

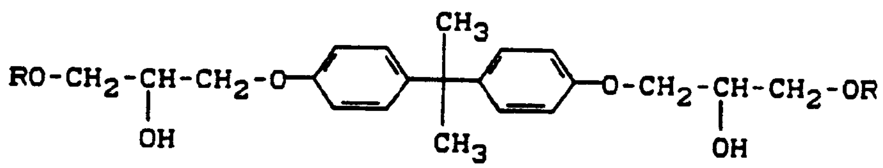

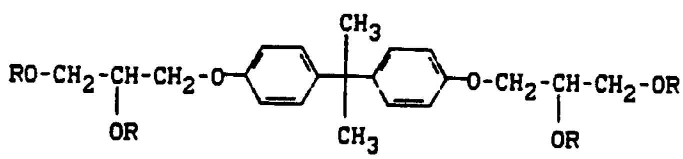

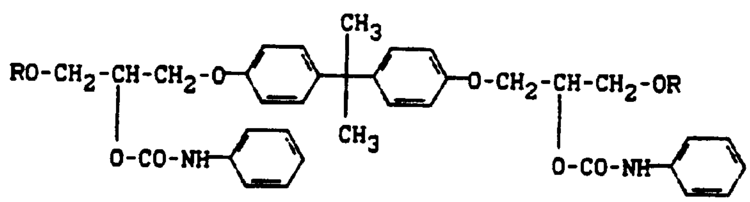

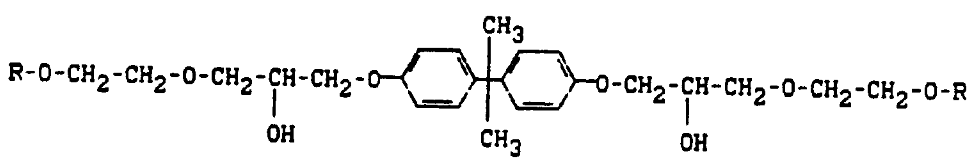

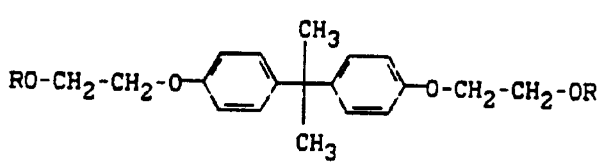

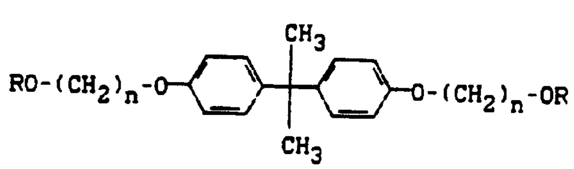

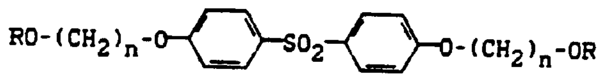

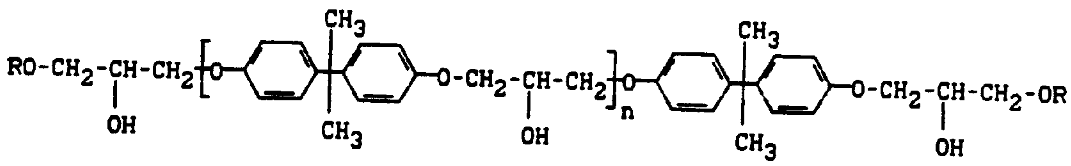

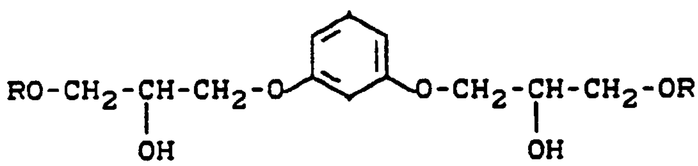

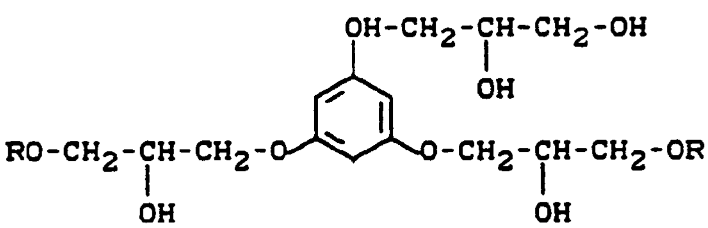

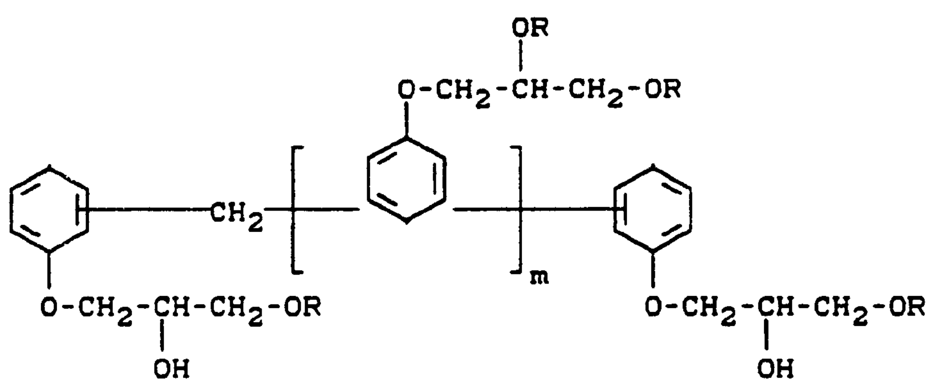

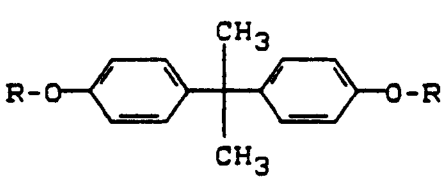

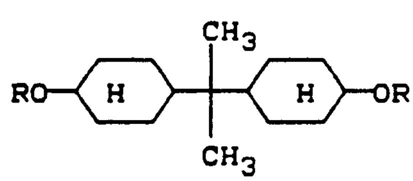

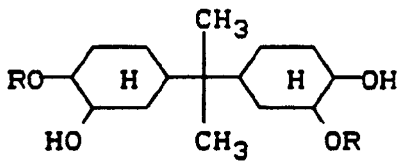

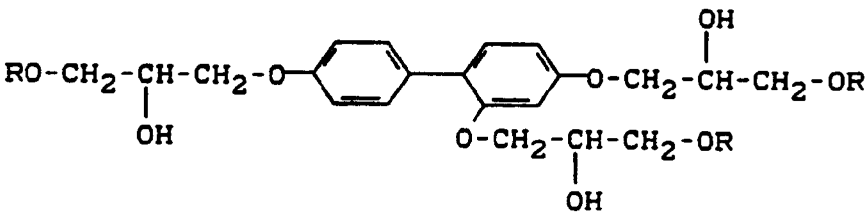

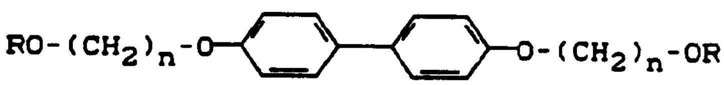

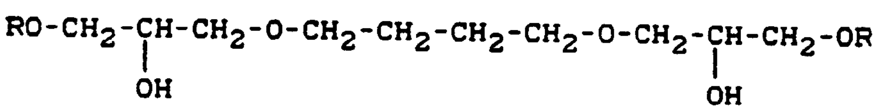

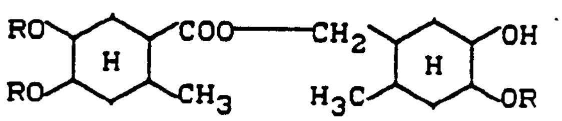

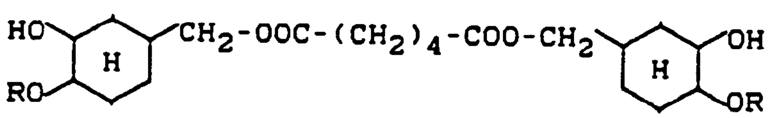

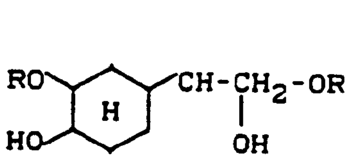

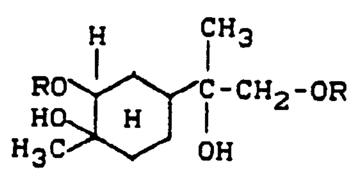

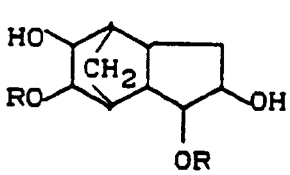

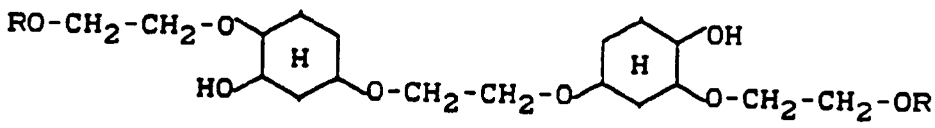

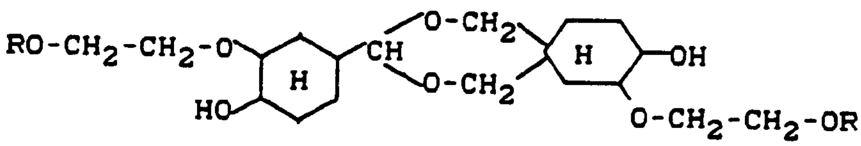

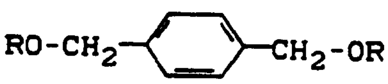

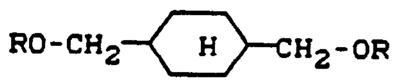

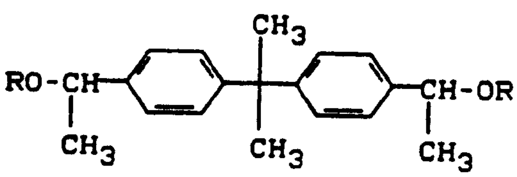

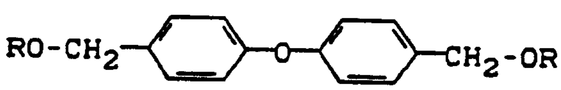

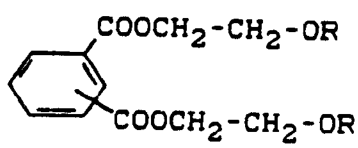

in der ortho-, meta- oder para-Form

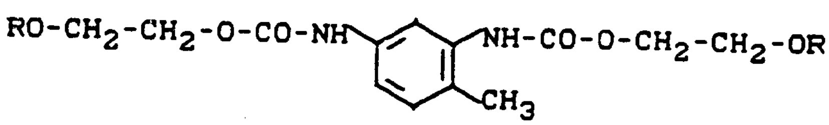

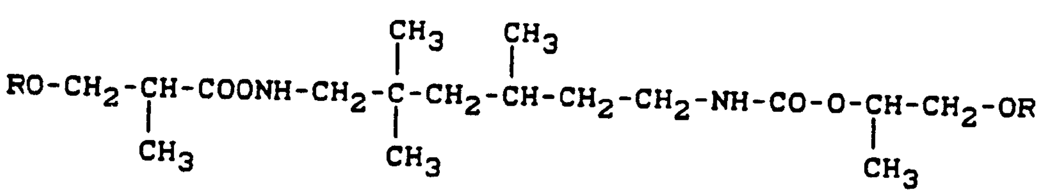

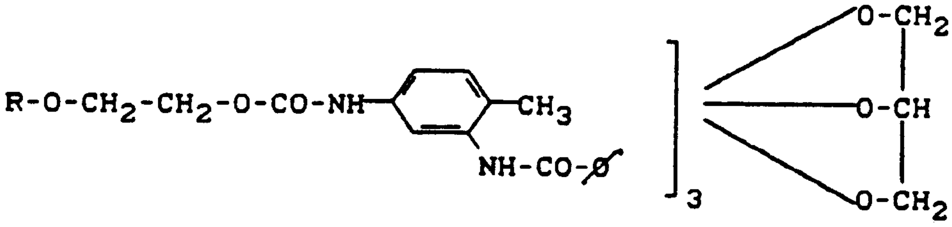

worin

R für 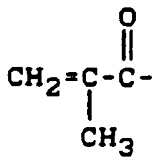 oder 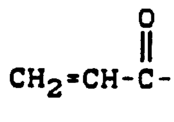

steht,

n eine Zahl von 1 bis 4 und

m eine Zahl von 0 bis 5 bedeutet.

Außerdem seien Derivate des Tricyclodecans (EP-A 00 23 686) und Umsetzungsprodukte aus Polyolen, Diisocyanaten und Hydroxyalkylmethacrylaten (DE-A 37 03 120, DE-A 37 03 080 und DE-A 37 03 130) genannt. Beispielsweise seien die folgenden Monomeren genannt:

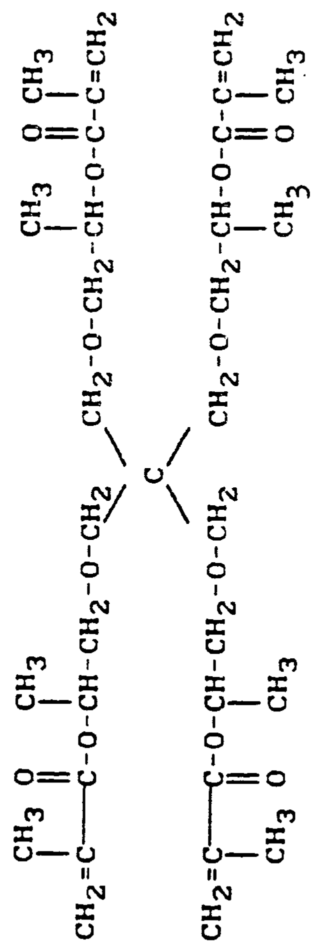

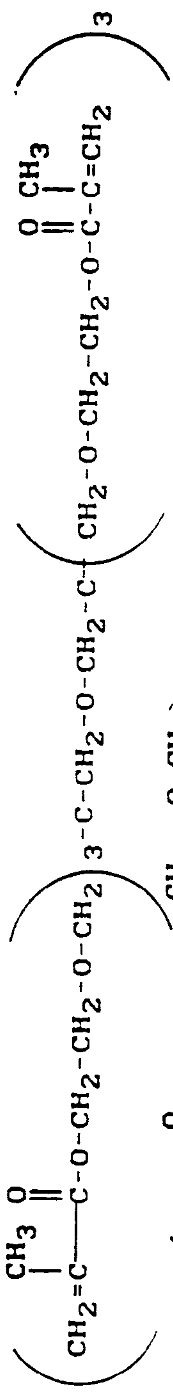

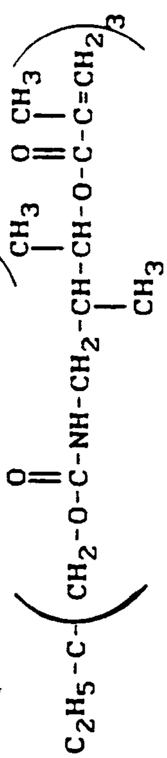

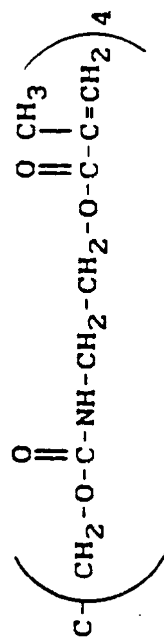

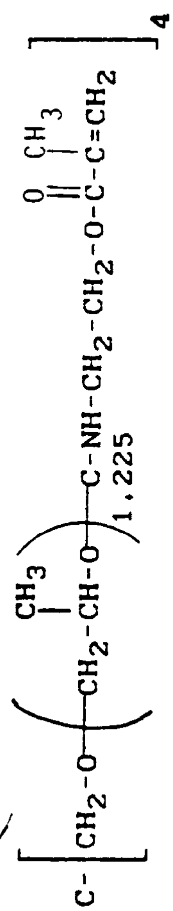

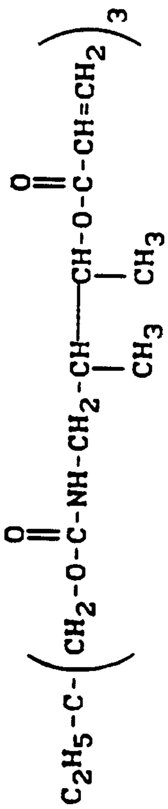

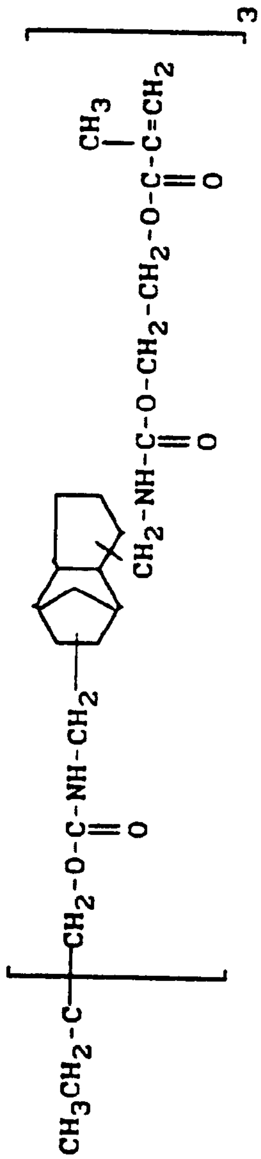

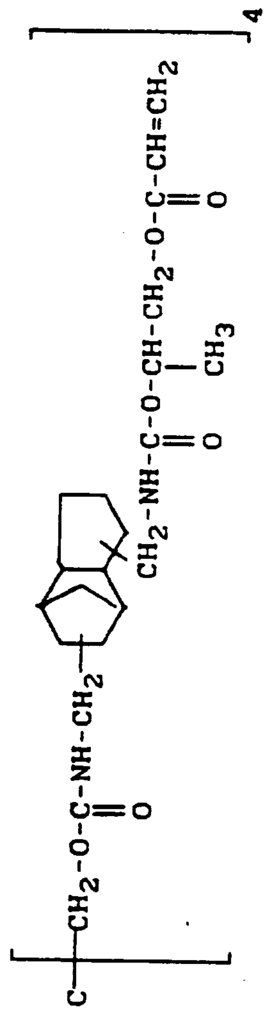

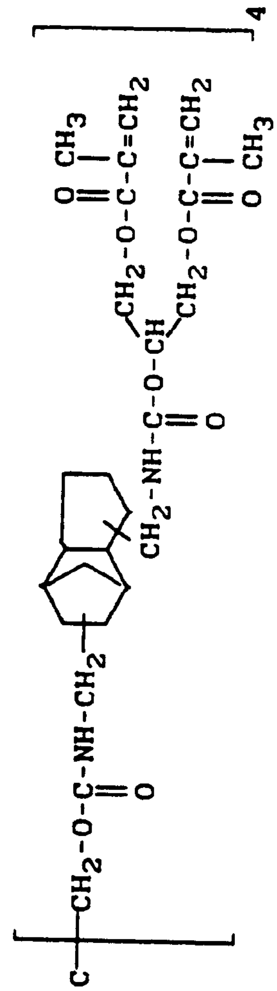

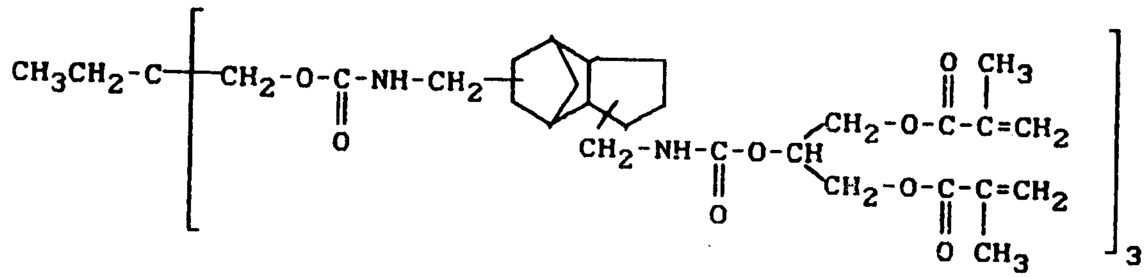

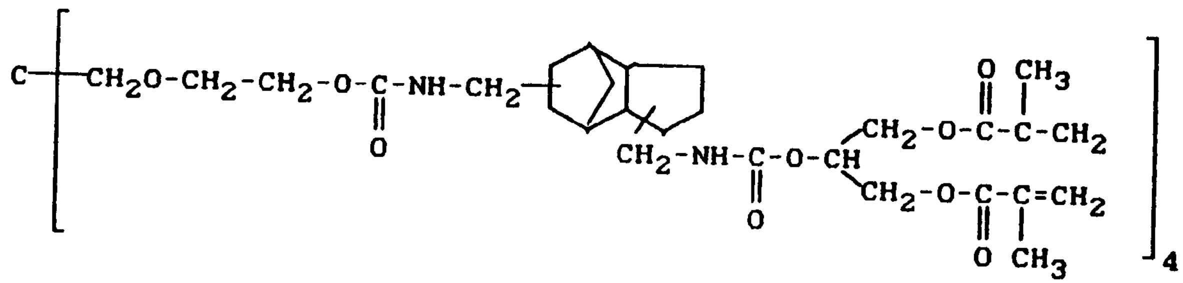

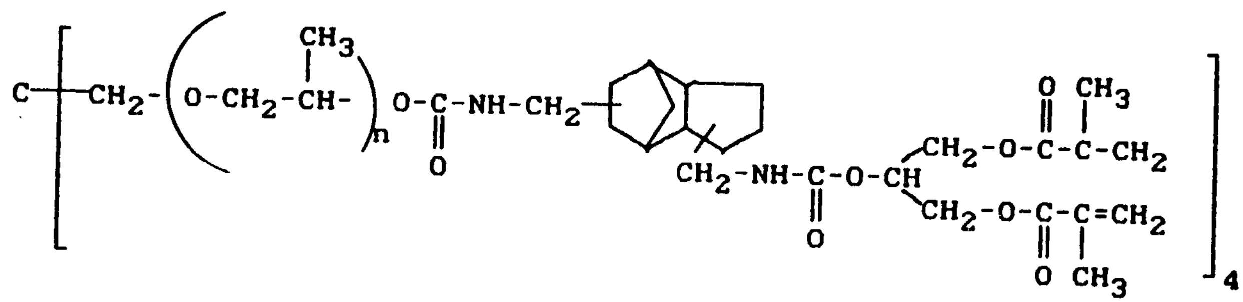

n = 1,225 (statistischer Mittelwert für 4 Ketten)

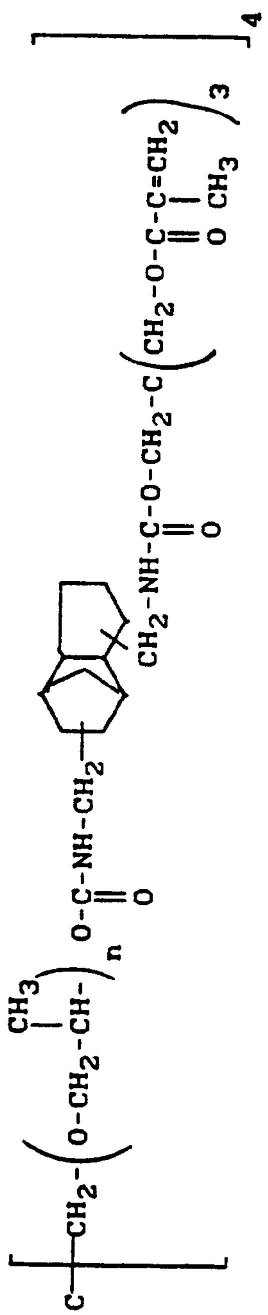

n = 1,225 (Mittelwert)

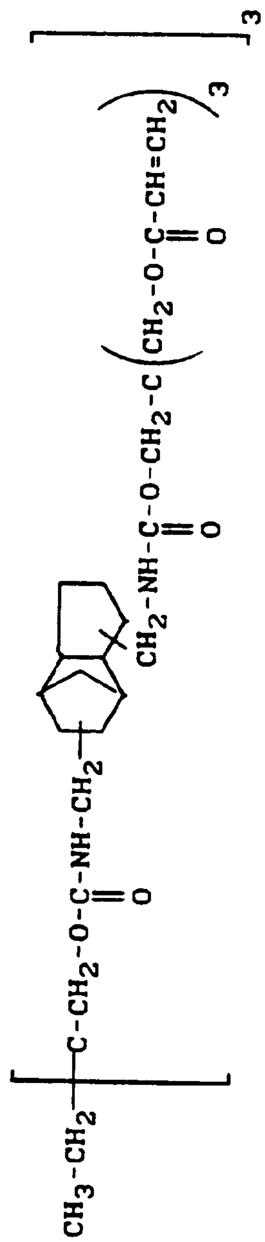

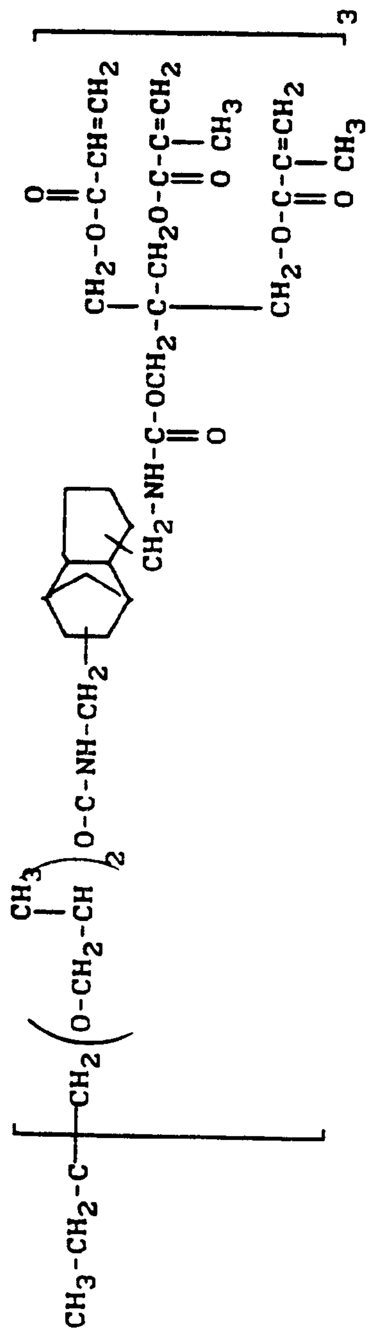

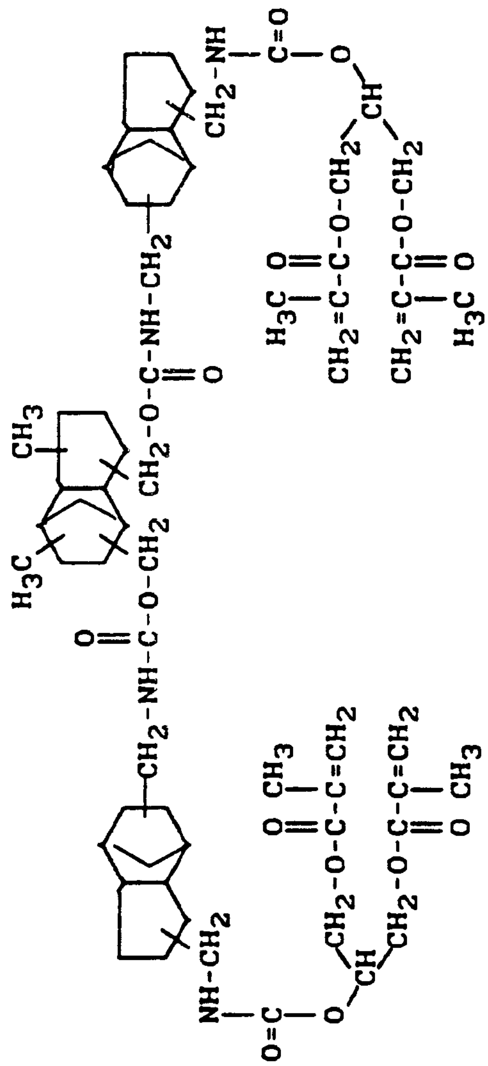

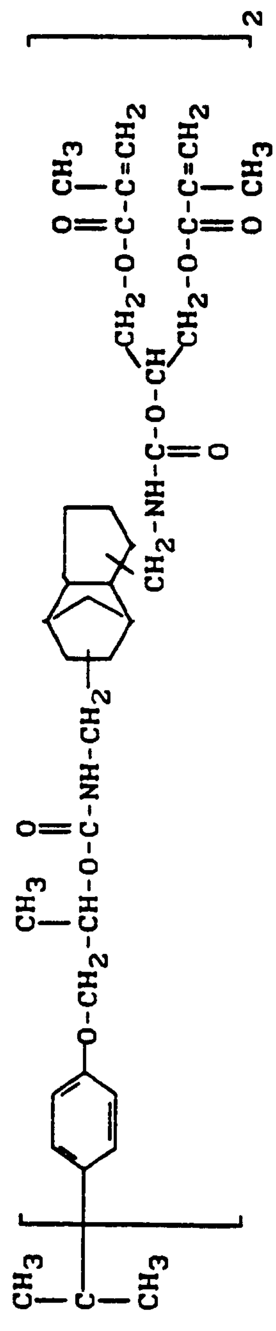

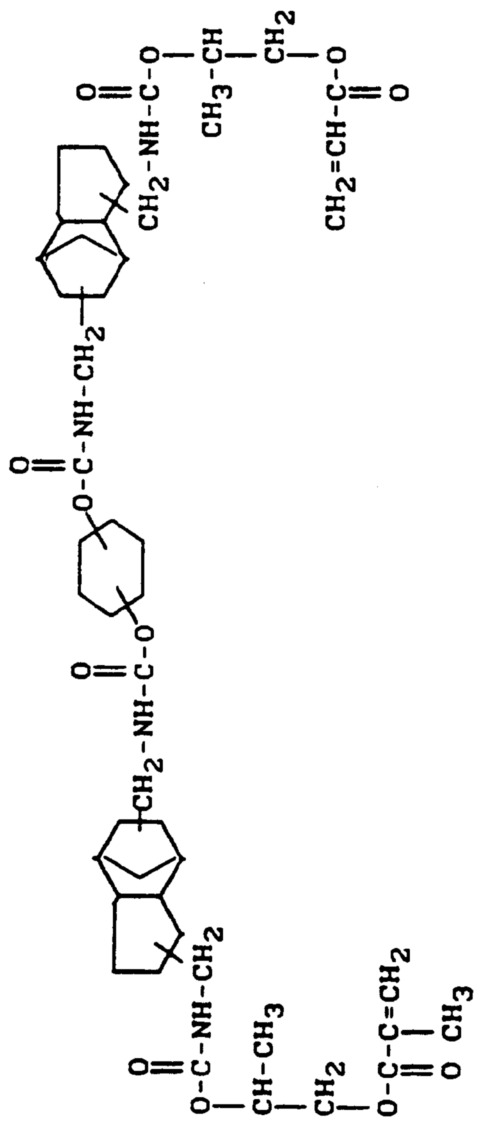

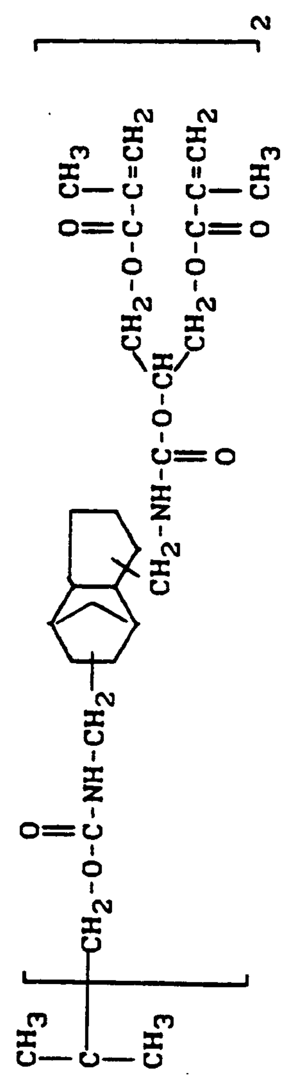

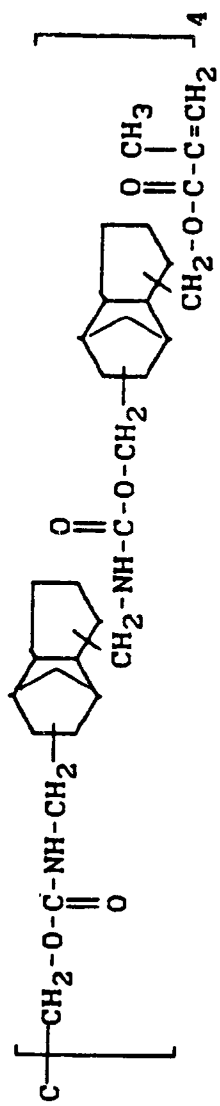

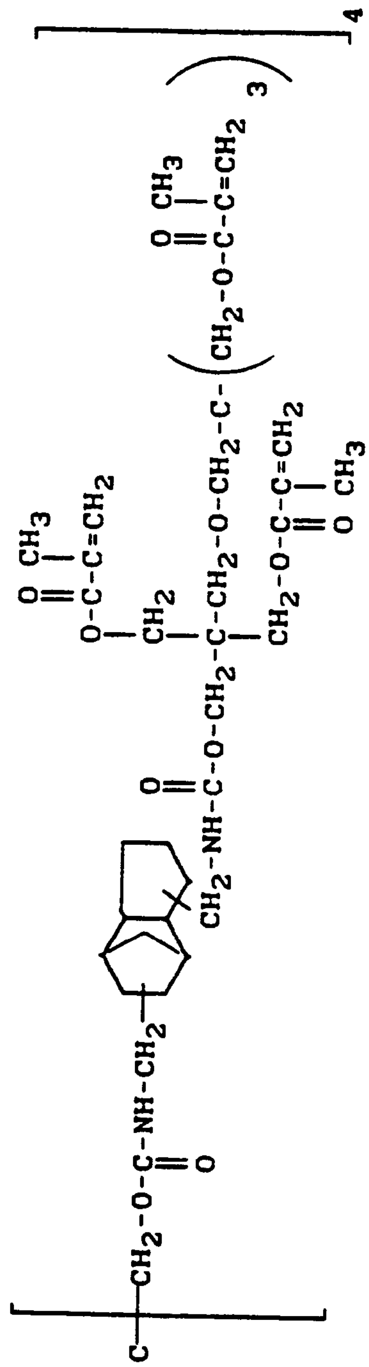

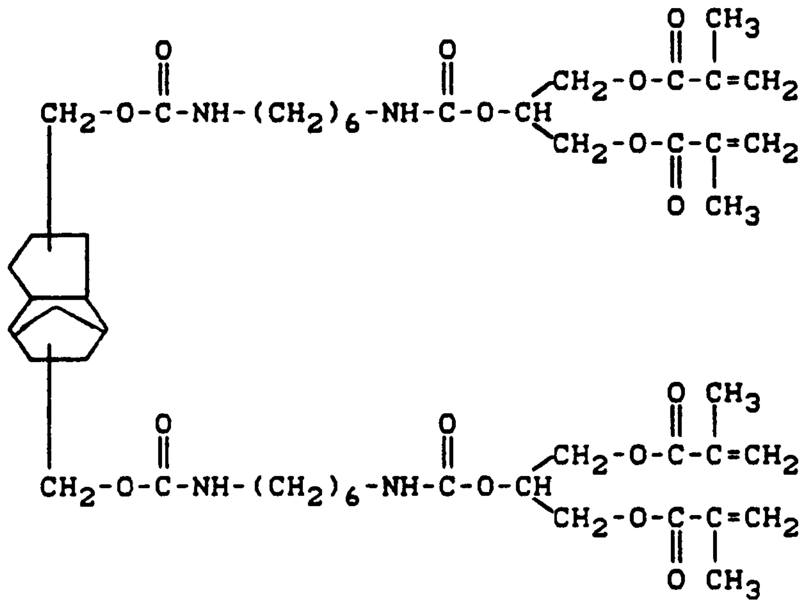

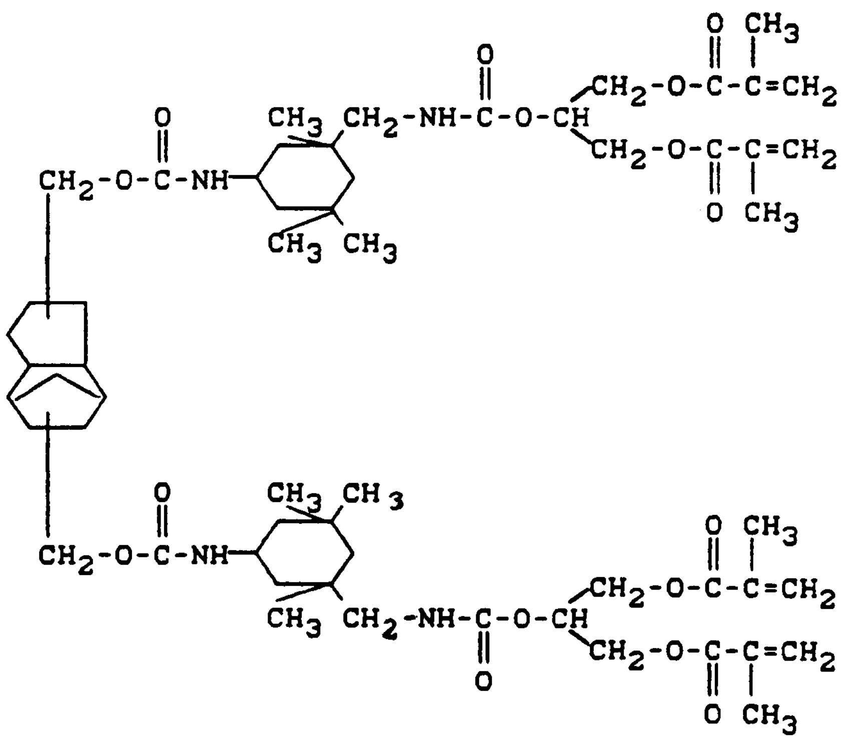

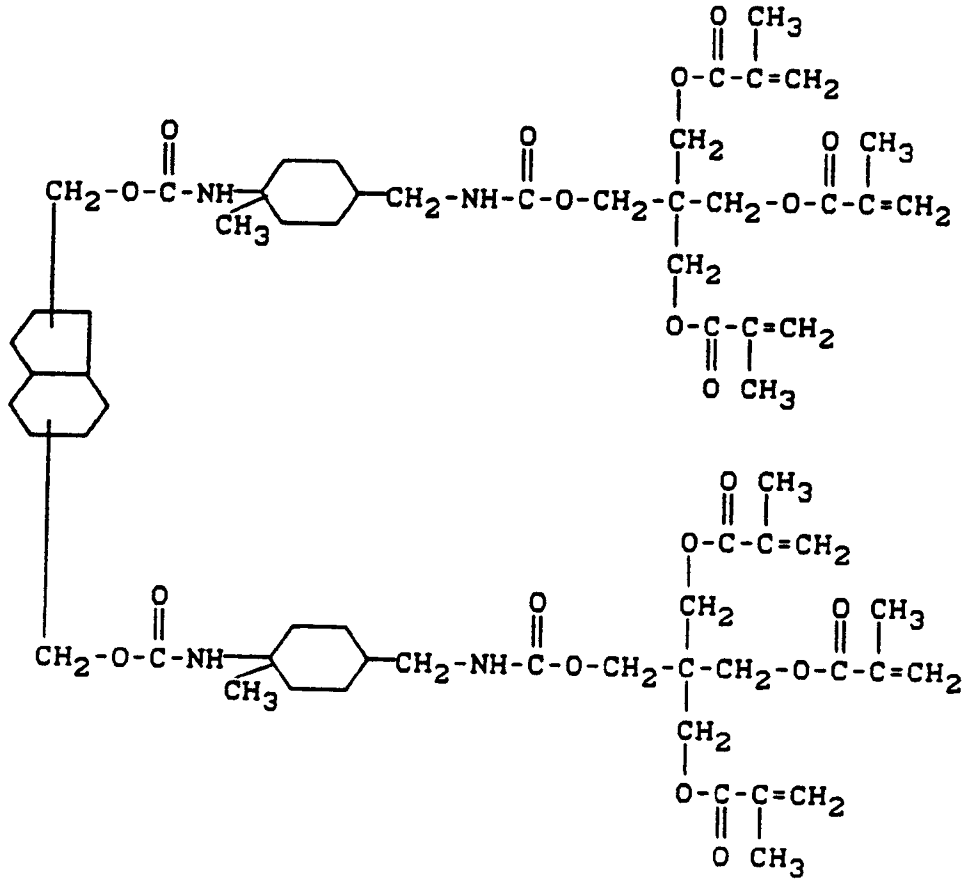

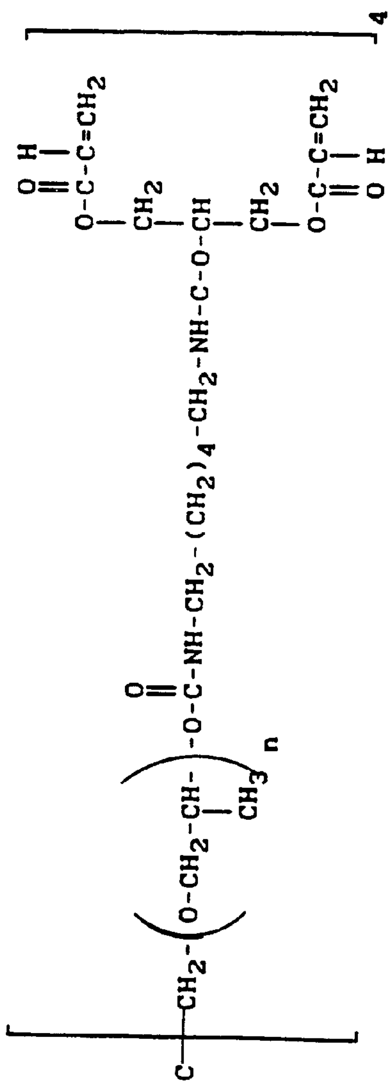

n = 1,225 (statistischer Mittelwert für 4 Ketten)

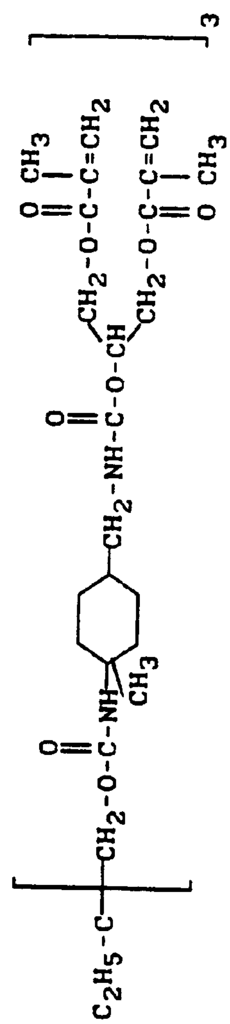

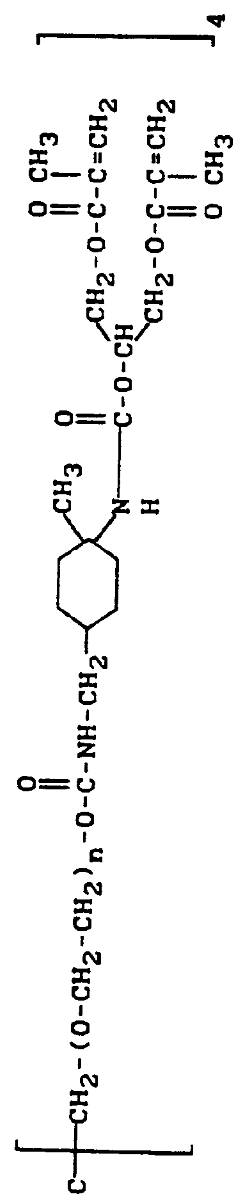

n = 1,225 (statistischer Mittelwert für 4 Ketten)

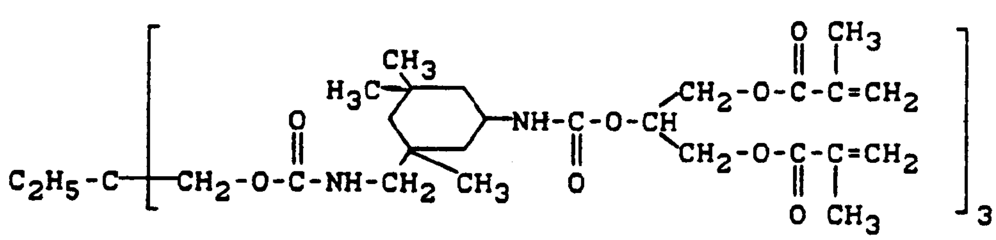

Besonders bevorzugt als Monomer wird das sogenannte Bis-GMA der Formel

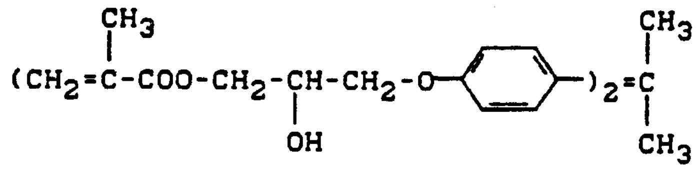 .

Selbstverständlich ist es möglich, Mischungen der verschiedenen (Meth)-Acrylsäureester, die Vernetzungen ausbilden können, einzusetzen. Beispielsweise seien Mischungen von 20 bis 70 Gew.-Teilen Bis-GMA und 30 bis 80 Gew.-Teilen Triethylenglykoldimethacrylat genannt.

Komponente (c)

(Meth)-Acrylsäureester, die keine Vernetzungen ausbilden können, sind im allgemeinen monofunktionelle (Meth)-Acrylate. Beispielsweise seien $C_1$-$C_{12}$-, vorzugsweise $C_1$-$C_4$-Alkylmethacrylate wie Methylmethacrylat, Ethylmethacrylat, n-Propylmethacrylat, i-Propylmethacrylat, n-Butylmethacrylat, tert.-Butylmethacrylat, Hydroxyalkylmethacrylate wie 2-Hydroxyethylmethacrylat, 2-Hydroxypropylmethacrylat, Diethylenglykolmonomethacrylat und Triethylenglykolmonomethacrylat und Alkoxyalkylmethacrylate wie 2-Methoxyethylmethacrylat, 3-Methoxybutylmethacrylat und Ethyltriglykolmethacrylat genannt.

Bevorzugte (Meth)-Acrylsäureester, die keine Vernetzungen ausbilden können, sind Methylmethacrylat, Ethylmethacrylat, Hydroxyethylmethacrylat.

Im allgemeinen setzt man als Monomermischung für die erfindungsgemäßen Dentalwerkstoffe die Komponenten (b) und (c) in Mischung ein. Diese Mischungen haben bevorzugt eine Viskosität im Bereich von 50 bis 5000 mPa.s, vorzugsweise in Bereich von 100 bis 2000 mPa.s (jeweils bei 20°C).

Komponente (d)

Die erfindungsgemäßen Dentalwerkstoffe können an sich bekannte Additive enthalten. Als Additive seien Starterzusätze, Stabilisatoren, Füllstoffe, Pigmente, Farbstoffe, Lichtschutzmittel, Fluoreszenzmittel oder Weichmacher genannt.

Als Starterzusätze für die Einleitung der Polymerisation können an sich bekannte Startersysteme (Literatur Houben Weyl, Methoden der organischen Chemie Band E20, S. 15 ff., Georg Thieme Verlag, Stuttgart 1987) verwendet werden. Es handelt sich um radikale, Anionen oder Kationen liefernde Systeme, die eine radikalische, anionische oder kationische Polymerisation auslösen können. Im Falle von Radikale lieferndem System sind Peroxide oder aliphatische Azoverbindungen besonders geignet, bei spielsweise Benzoylperoxid, Laurylperoxid oder Azoisobuttersäuredinitril; die Systeme werden üblicherweise in Mengen von 0,1 bis 5 Gew.-% eingesetzt. Während die Härtung bei erhöhter Temperatur allein durch Peroxide oder andere Radikalstarter durchgeführt werden kann, ist zur Härtung bei Raumtemperatur im allgemeinen ein Zusatz von Beschleunigern, vorzugsweise aromatischen Aminen, zweckmäßig. Geeignete Beschleuniger sind z.B. N,N-substituierte Toluidine und Xylidine, wie N,N-Dimethyl-p-toluidin oder N,N-Bis-(2-Hydroxy-ethyl)-xylidin. Die Aushärtung kann im allgemeinen durch Zusatz von 0,5 bis 3 Gew.-% der genannten Amine erreicht werden.

Es ist jedoch auch möglich, Dentalwerkstoffe herzustellen, die unter Einwirkung von Licht, beispielsweise UV-Licht, sichtbarem Licht oder Laserlicht, polymerisieren. In diesen Fällen setzt man Fotopolymerisationsinitiatoren und Beschleuniger ein.

Fotopolymerisationsinitiatoren sind an sich bekannt (Literatur Houben Weyl, Methoden der organischen Chemie, Band E20, S. 80 ff, Georg Thieme Verlag Stuttgart 1987). Vorzugsweise handelt es sich dabei um Carbonylverbindungen, wie Benzoin und dessen Derivate, insbesondere Benzoinmethylether, Benzoyl und Benzylderivate, beispielsweise 4,4-Oxidibenzyl und andere Dicarbonylverbindungen wie Thiacetyl, 2,3-Pentadion oder Metallcarbonyle wie Pentacarbonylmangan, Chinone wie 9,10-Phenanthrenchinon und Campherchinon oder deren Derivate.

Der Anteil an solchen Fotopolymerisationsinitiatoren beträgt vorzugsweise etwa 0,01 bis etwa 5 Gew.-% der Gesamtzusammensetzung.

Die mit Licht härtbaren fotopolymerisierbaren Massen enthalten vorzugsweise noch Substanzen, die in Gegenwart von Fotopolymerisationsinitiatoren die Polymerisationsreaktion beschleunigen. Bekannte Beschleuniger sind beispielsweise aromatische Amine wie p-Toluidin und Dimethyl-p-toluidin, Trialkylamine wie Trihexylamin, Polyamine wie N,N,N',N'-Tetraalkylalkylendiamin, Barbitursäure und Dialkylbarbitursäure und

Sulfimide.

Die Beschleuniger werden im allgemeinen in einer Menge von 0,01 bis etwa 5 Gew.-% der Gesamtmischung eingesetzt.

Es ist auch möglich, den erfindungsgemäßen Dentalwerkstoffen UV-Stabilisatoren zuzusetzen, um das Nachdunkeln während des Alterns zu vermeiden.

Ein besonders geeigneter UV-Stabilisator ist 2-Hydroxy-4-methoxybenzophenon. Ein weiteres bevorzugtes Material ist 2-(2'-Hydroxy-5-methylphenyl)-benzotriazol. Beispielsweise seien noch Hydrochinon, p-Benzochinon und p-Butylhydroxytoluol genannt.

Zur Einstellung einer möglichst naturgetreuen Farbe können die erfindungsgemäßen Dentalwerkstoffe auch an sich bekannte Pigmente und Farbstoffe enthalten.

Insbesondere bevorzugt werden Dentalwerkstoffe, enthaltend

a) 10 bis 30 Gew.-Teile eines Füllstoffs, enthaltend polymere vernetzte (Meth)-Acrylate mit einer Teilchengröße im Bereich von 0,01 bis 10 µm, einem Quellungsgrad von 50 bis 2000 Gew.-% und einem Vernetzungsgrad von 50 bis 100 Gew.-%, jeweils bezogen auf das Polymer,

b) 50 bis 90 Gew.-Teile (Meth)-Acrylate, die Vernetzungen ausbilden können,

c) 0 bis 40 Gew.-Teile (Meth)-Acrylate, die keine Vernetzungen ausbilden können

und

d) 0,1 bis 10 Gew.-Teile eines oder meherer Additive.

Es wurde auch ein Verfahren zur Herstellung der erfindungsgemäßen Dentalwerkstoffe gefunden, das dadurch gekennzeichnet ist, daß man eine Mischung aus

a) 5 bis 35 Gew.-Teile eines Füllstoffes enthaltend polymere vernetzte (Meth)-Acrylate mit einer Teilchengröße im Bereich von 0,01 bis 10 µm, einem Quellungsgrad von 50 bis 2000 Gew.-% und einem Vernetzungsgrad von 50 bis 100 Gew.-%, jeweils bezogen auf das Polymer,

b) 40 bis 90 Gew.-Teilen (Meth)-Acrylate, die Vernetzungen ausbilden können,

c) 0 bis 40 Gew.-Teilen (Meth)-Acrylate, die keine Vernetzungen ausbilden können und

d) 0,1 bis 10 Gew. -Teilen Additive

polymerisiert.

Die Polymerisation wird im allgemeinen unter den oben genannten Bedingungen ausgeführt.

Die erfindungsgemäßen Dentalwerkstoffe können unter Formgebung zu künstlichen Zähnen und Zahnersatzteilen wie Kronen, Brücken, Inlays und Onlays, zu Zahnfüllungen und Zahnlacken verarbeitet werden. Die polymerisierten, erfindungsgemäßen Dentalwerkstoffe zeichnen sich durch eine günstige Eigenschaftskombination aus. Sie besitzen eine hohe Härte, eine hohe Steifigkeit und eine hohe Abrasionsbeständigkeit bei guter Zähigkeit. Sie können leicht eingefärbt werden und der natürlichen Zahnfarbe angepaßt werden.

Beispiel 1

Herstellung eines Polymethacrylsäureesters aus Ethylenglykoldimethacrylat

In einem 3-Liter-Glasreaktor, der mit Blattrührer, Rückflußkühler, Innenthermometer, Gaseinlaß und Gasauslaßrohr ausgerüstet ist, werden 1800 g Butanon-2, 200 g Ethylenglykoldimethacrylat und 2 g Dibenzoylperoxid eingewogen. Unter Rühren mit 300 UpM und unter Stickstoffspülung wird 2 Stunden am Rückfluß erhitzt. Es entsteht eine gut rührbare Suspension. Aus dieser lassen sich durch Sprühtrocknung 190 g feines Pulver gewinnen. Die mittlere Teilchengröße (gemessen mit Laserkorrelationsspektroskopie) beträgt 700 nm, Gelgehalt 98,4 % und der Quellungsgrad (gemessen in THF) 310 %.

Beispiel 2

Herstellung eines Polymethacrylsäureesters aus Glycerindimethacrylat

Nach der in Beispiel 1 angegebenen Arbeitsweise wurden 500 g Glycerindimethacrylat und 5 g Dibenzoylperoxid in 2000 g Butanon-2 umgesetzt. Man erhält 475 g Pulver mit einer Teilchengröße von 350 nm, einem Gelgehalt von 97,3 % und einem Quellungsgrad von 280 %.

Beispiel 3

Polymerisierbare Masse

104 g Polymerisat aus Beispiel 1, 248 g Bis-GMA, 152 g Triethylenglykoldimethacrylat und 2,1 g

Dibenzoylperoxid werden in einem Laborkneter in 30 Min. verknetet. Die erhaltene Masse wird 5 Stunden bei 35°C gelagert. Man erhält eine transparente, nicht Klebende, teigartige Paste.

Beipiel 4

Polymerisierbare Masse

36 g Polymerisat aus Beispiel 2, 120 g des Umsetzungsproduktes aus 2,2,4-Trimethylhexamethylendiisocyanat und 2 Mol 2-Hydroxyethylmethacrylat [1,6-Bis-(Methacryloyl-oxyethoxycarbonylamino)-2,2,4-trimethylhexan] und 0,64 g Dibenzoylperiod werden wie im Beispiel 3 beschrieben zu einer Masse verknetet.

Beispiel 5

20 g Polymerisat aus Beispiel 2, 60 g Bis-GMA, 30 g Triethylenglykodimethacrylat und 10 g 4-Methoxybutylmethacrylat und 0,5 g Dibenzoylperoxid werden wie in Beispiel 3 beschrieben zu einer Masse verknetet.

Beispiel 6

Die Massen aus den Beispielen 3, 4 und 5, sowie 2 Vergleichsmaterialien wurden bei 140°C und 200 bar in 10 Min. zu einer Prüfplatte polymerisiert. Es wurden Werte für die Biegefestigkeit nach DIN 13 922 und den Biege-E-Modul nach DIN 13 922 sowie die Eindringtiefe nach Wallace ermittelt.

Die Wallacemethode dient zur Bestimmung der Eindruckhärte an Kunststoffen. Ein Vickers-Diamant wird unter einer Vorlast von 1 p auf die Oberfläche aufgebracht und anschließend 60 sec lang mit einer Hauptlast von 100 p beansprucht. Als Maß für den Eindringwiderstand wird die Eindringtiefe des Diamanten unter Hauptlast gemessen. Im Gegensatz zu den Vickers- oder Brinellhärtemessungen, bei denen die Prüfkraft auf die Dimension des bleibenden Eindrucks bezogen wird, erfaßt man mit der Wallacemethode die elastische und die bleibende Verformung des Kunststoffes.

| | Biegefestigkeit $N/nm^2$ | E-Modul $N/nm^2$ | HW µm |
|---|---|---|---|
| Beispiel 3 | 140 ± 7 | 3800 ± 150 | 16,2 ± 0,9 |
| Beispiel 4 | 136,1 ± 11 | 4100 ± 70 | 15,8 ± 0,5 |
| Beispiel 5 | 132 ± 10 | 3850 ± 130 | 17,0 ± 0,6 |
| übliches PMMA-System | 112 ± 9 | 2800 ± 100 | 22,8 ± 1,5 |
| WO 82/02556 (US-A-4,396,377) Beispiel 1 | 118 ± 10 | 3050 ± 80 | 20,3 ±1,1 |

**Patentansprüche**

**Patentansprüche für folgende Vertragsstaaten : AT, BE, CH, DE, FR, GB, GR, IT, LI, LU, NL, SE**

1. Dentalwerkstoffe, enthaltend
   a) 5 bis 35 Gew.-Teile eines Füllstoffs, bestehend aus polymeren vernetzten (Meth)-Acrylaten mit einer Teilchengröße im Bereich von 0,01 bis 10 µm, einem Quellungsgrad von 50 bis 2000 Gew.-% und einem

Vernetzungsgrad von 50 bis 100 Gew.-%, jeweils bezogen auf das Polymer,
b) 40 bis 90 Gew.-Teile (Meth)-Acrylate, die Vernetzungen ausbilden können,
c) 0 bis 40 Gew.-Teile (Meth)-Acrylate, die keine Vernetzungen ausbilden können und
d) 0,1 bis 10 Gew.-Teile eines oder mehrerer Additive.

**2.** Dentalwerkstoff nach Anspruch 1, dadurch gekennzeichnet, daß der Füllstoff einen Gelgehalt von 90 bis 100 Gew.-% aufweist.

**3.** Dentalwerkstoff nach den Ansprüchen 1 und 2, dadurch gekennzeichnet, daß der Füllstoff eine aktive Oberfläche von 20 bis 600 $m^2$ /g aufweist.

**4.** Dentalwerkstoff nach den Ansprüchen 1 bis 3, dadurch gekennzeichnet, daß die Mischung der (Meth)Acrylsäureester, die Vernetzungen und keine Vernetzungen ausbilden können, eine Viskosität im Bereich von 50 bis 5000 mPa.s aufweist.

**5.** Verfahren zur Herstellung von Dentalwerkstoffen gemäß Ansprüchen 1 - 4, dadurch gekennzeichnet, daß man eine Mischung aus
a) 5 bis 35 Gew.-Teilen eines Füllstoffes enthaltend polymere vernetzte (Meth)-Acrylate mit einer Teilchengröße im Bereich von 0,01 bis 10 µm, einem Quellungsgrad von 50 bis 2000 Gew.-% und einem Vernetzungsgrad von 50 bis 100 Gew.-%, jeweils bezogen auf das Polymer,
b) 40 bis 90 Gew.-Teilen (Meth)-Acrylate, die Vernetzungen ausbilden können, und
c) 0 bis 40 Gew.-Teilen (Meth)-Acrylate, die keine Vernetzungen ausbilden können und
d) 0,1 bis 10 Gew.-Teilen Additive
polymerisiert.

**6.** Verwendung von polymerisierten Dentalwerkstoffen nach den Ansprüchen 1 bis 4 zur Herstellung von künstlichen Zähnen, Zahnersatzteilen, Zahnfüllungen und Zahnlacken.

**Patentansprüche für folgenden Vertragsstaat : ES**

**1.** Verfahren zur Herstellung von Dentalwerkstoffen, enthaltend
a) 5 bis 35 Gew.-Teile eines Füllstoffes, bestehend aus polymeren vernetzten (Meth)-Acrylaten mit einer Teilchengröße im Bereich von 0,01 bis 10 µm, einem Quellungsgrad von 50 bis 2000 Gew.-% und einem Vernetzungsgrad von 50 bis 100 Gew.-%, jeweils bezogen auf das Polymer,
b) 40 bis 90 Gew.-Teile (Meth)-Acrylate, die Vernetzungen ausbilden können, und
c) 0 bis 40 Gew.-Teile (Meth)-Acrylate, die keine Vernetzungen ausbilden können und
d) 0,1 bis 10 Gew.-Teile Additive,
dadurch gekennzeichnet, daß man eine Mischung aus den komponenten a) bis d) polymerisiert.

## Claims

**Claims for the following Contracting States : AT, BE, CH, DE, FR, GB, GR, IT, LI, LU, NL, SE**

**1.** Dental materials containing
a) 5 to 35 parts by weight of a filler consisting of polymeric crosslinked (meth)-acrylates having a particle size in the range from 0.01 to 10 µm, a degree of swelling of 50 to 2,000 % by weight and a degree of crosslinking of 50 to 100 % by weight, in each case based on the polymer,
b) 40 to 90 parts by weight of (meth)-acrylates which can form crosslinkages,
c) 0 to 40 parts by weight of (meth)-acrylates which cannot form crosslinkages and
d) 0.1 to 10 parts by weight of one or more additives.

**2.** Dental material according to Claim 1, characterised in that the filler has a gel content of 90 to 100 % by weight.

**3.** Dental material according to Claims 1 and 2, characterised in that the filler has an active surface area of 20 to 600 $m^2$/g.

**4.** Dental material according to Claims 1 to 3, characterised in that the mixture of (meth)-acrylic acid esters

which can form crosslinkages and no crosslinkages has a viscosity in the range from 50 to 5,000 mPa.s.

5. Process for the preparation of dental materials according to Claims 1 to 4, characterised in that a mixture of
   a) 5 to 35 parts by weight of a filler consisting of polymeric crosslinked (meth)-acrylates having a particle size in the range from 0.01 to 10 μm, a degree of swelling of 50 to 2,000 % by weight and a degree of crosslinking of 50 to 100 % by weight, in each case based on the polymer,
   b) 40 to 90 parts by weight of (meth)-acrylates which can form crosslinkages,
   c) 0 to 40 parts by weight of (meth)-acrylates which cannot form crosslinkages and
   d) 0.1 to 10 parts by weight of additives, is polymerized.

6. Use of polymerized dental materials according to Claims 1 to 4 for the production of false teeth, dental prostheses, dental fillings and dental lacquers.

**Claims for the following Contracting State : ES**

1. Process for the preparation of dental materials containing.
   a) 5 to 35 parts by weight of a filler consisting of polymeric crosslinked (meth)-acrylates having a particle size in the range from 0.01 to 10 μm, a degree of swelling of 50 to 2,000 % by weight and a degree of crosslinking of 50 to 100 % by weight, in each case based on the polymer,
   b) 40 to 90 parts by weight of (meth)-acrylates which can form crosslinkages,
   c) 0 to 40 parts by weight of (meth)-acrylates which cannot form crosslinkages and
   d) 0.1 to 10 parts by weight of additives,
   characterised in that a mixture of the components a) to d) is polymerized

**Revendications**

**Revendications pour les Etats contractants suivants : AT, BE, CH, DE, FR, GB, GR, IT, LI, LU, NL, SE**

1. Matériaux dentaires, contenant
   a) 5 à 35 parties en poids d'une charge, constituée de (méth)-acrylates polymériques réticulés ayant un diamètre de particules dans la plage de 0,01 à 10 μm, un degré de gonflement de 50 à 2000 % en poids et un degré de réticulation de 50 à 100 % en poids, dans chaque cas par rapport au polymère,
   b) 40 à 90 parties en poids de (méth)-acrylates qui peuvent créer des réticulations,
   c) 0 à 40 parties en poids de (méth)-acrylates qui ne peuvent pas créer de réticulations et
   d) 0,1 à 10 parties en poids d'un ou plusieurs additifs.

2. Matériau dentaire suivant la revendication 1, caractérisé en ce que la charge présente une teneur en gel de 90 à 100 % en poids.

3. Matériau dentaire suivant les revendications 1 et 2, caractérisé en ce que la charge présente une surface active de 20 à 600 $m^2/g$.

4. Matériau dentaire suivant les revendications 1 à 3, caractérisé en ce que le mélange des esters d'acide (méth)-acrylique qui peuvent créer des réticulations et de ceux qui ne peuvent pas en créer présente une viscosité dans la plage de 50 à 5000 mPa.s.

5. Procédé de production de matériaux dentaires suivant les revendications 1 à 4, caractérisé en ce qu'on polymérise un mélange
   a) de 5 à 35 parties en poids d'une charge contenant des (méth) -acrylates polymériques réticulés ayant un diamètre de particules dans la plage de 0,01 à 10 μm, un degré de gonflement de 50 à 2000 % en poids et un degré de réticulation de 50 à 100 % en poids, dans chaque cas par rapport au polymère,
   b) de 40 à 90 parties en poids de (méth)-acrylates qui peuvent créer des réticulations et
   c) de 0 à 40 parties en poids de (méth)-acrylates qui ne peuvent pas créer de réticulations et
   d) de 0,1 à 10 parties en poids d'additifs.

6. Utilisation de matériaux dentaires polymérisés suivant les revendications 1 à 4 pour la réalisation de dents artificielles, de parties de prothèses dentaires, d'obturations dentaires et de laques dentaires.

**Revendications pour l'Etat contractant suivant : ES**

1. Procédé de production de matériaux dentaires, contenant
   a) 5 à 35 parties en poids d'une charge constituée de (méth)-acrylates polymériques réticulés ayant un diamètre de particules dans la plage de 0,01 à 10 µm, un degré de gonflement de 50 à 2000 % en poids et un degré de réticulation de 50 à 100 % en poids, dans chaque cas par rapport au polymère,
   b) 40 à 90 parties en poids de (méth)-acrylates qui peuvent créer des réticulations et
   c)0 à 40 parties en poids de (méth)-acrylates qui ne peuvent pas créer de réticulations et
   d) 0,1 à 10 parties en poids d'additifs,

   caractérisé en ce qu'on polymérise un mélange des composants a) à d).